# FASCICULE DE BREVET EUROPEEN

(11) **EP 0 755 281 B1**
(45) Date de publication et mention de la délivrance du brevet: **18.03.1998**
(21) Numéro de dépôt: 95917382.4
(22) Date de dépôt: 12.04.1995
(51) Int. Cl.: A61M 29/02

(54) **CATHETER DE DILATATION A ECHANGE RAPIDE**
DILATATIONSKATHETER ZUM SCHNELLEN AUSWECHSELN
RAPID EXCHANGE INFLATABLE CATHETER

(30) Priorité: 15.04.1994 FR 9404497
(43) Date de publication de la demande: 29.01.1997
(73) Titulaire: LABORATOIRES NYCOMED S.A., 75644 Paris Cédex 13 (FR)
(72) Inventeur: HILAIRE, Pierre, F-75009 Paris (FR); LAGARDE, Vincent, F-75012 Paris (FR)
(74) Mandataire: Hubert, Philippe
(86) Numéro de dépôt international: FR9500472
(87) Numéro de publication internationale: WO9528197

(56) Documents cités:
- WO-A-94/04216

## Description

La présente invention a pour objet un cathéter de dilatation à échange rapide, destiné à être introduit dans un canal corporel tel que notamment un vaisseau sanguin.

L'invention trouve principalement application dans le domaine du traitement des affections des artères coronaires mais peut être également utilisée dans le domaine du traitement des affections d'autres canaux corporels, comme par exemple l'oesophage ou l'urètre.

Ces affections résultent généralement de la présence, sur les parois internes du canal, de dépôts entraînant des rétrécissements ou sténoses de celui-ci.

Dans le traitement de ce type d'affections, un cathéter de dilatation est généralement utilisé pour rétablir la section normale de passage du canal au niveau de la sténose par compression à l'aide d'un ballon (ou ballonnet).

Un guide, habituellement réalisé sous forme d'un fil métallique est utilisé pour faciliter la progression du cathéter jusqu'à la sténose.

Le fil-guide est généralement plus long que le cathéter (de l'ordre de 20 à 50 cm) pour permettre l'avancement du cathéter à l'intérieur du canal corporel, par glissement le long du fil.

Au cours d'une intervention utilisant un cathéter de dilatation, il peut être nécessaire de changer le cathéter en laissant éventuellement le fil-guide en position, par exemple en cas de difficulté de progression à l'intérieur des coronaires.

On connaît notamment par le brevet US 4.748.982 un cathéter dit "à échange rapide" dont la partie distale comprend deux canaux internes non communiquant juxtaposés, dont l'un (destiné au passage du fil-guide) débouche à l'extérieur du cathéter.

Dans la mesure où ce cathéter n'est traversé par le fil-guide que sur une faible portion de sa longueur (20 à 25 cm pour un cathéter d'une longueur totale d'environ 130 cm) inférieure à la longueur du fil s'étendant à l'extérieur du corps (35 à 55 cm), il est possible de le retirer en maintenant ledit fil en position et en le faisant glisser le long du fil-guide.Il est ainsi possible de remplacer le cathéter initial par un autre cathéter, sans faire appel à un fil d'échange spécifique ou à une rallonge.

On connaît encore par le document WO 94/04216 un cathéter de dilatation à échange rapide, destiné à être introduit dans un canal corporel tel que notamment un vaisseau sanguin, comprenant :
- un corps tubulaire flexible comprenant une partie distale, une partie intermédiaire et une partie proximale, et comportant :
- une portion déformable radialement formant ballon, disposée au niveau de sa partie distale ;
- un premier conduit interne débouchant à une extrémité à l'intérieur du ballon, de façon étanche, et reliée à l'autre extrémité à une source d'alimentation en fluide pour permettre le gonflage et le dégonflage du ballon ;
- un second conduit interne, non communiquant avec ledit premier conduit interne, traversant ladite partie distale, et comportant une ouverture au niveau de la partie intermédiaire et au voisinage de la partie proximale, ledit conduit étant défini par une paroi sensiblement tubulaire et adapté pour permettre le passage d'un fil-guide sortant dudit second conduit interne par ladite ouverture, ledit corps comportant en outre une âme présentant un module élastique d'au moins 10000 Mpa reliée fixement à une extrémité à la partie proximale et noyée à son autre extrémité dans la paroi précitée définissant ledit second conduit interne.

Pour positionner correctement le ballon au niveau de la sténose, il est nécessaire d'amener l'extrémité distale du cathéter au-delà de ladite sténose.

Comme on le comprend, le franchissement de la sténose par l'extrémité distale du cathéter nécessite généralement l'application sur ce dernier d'une poussée.

Cette poussée est exercée par le praticien au niveau de l'extrémité proximale du cathéter.

La transmission de cette poussée jusqu'à l'extrémité distale du cathéter soulève un problème qui n'a pas été résolu jusqu'à ce jour d'une manière satisfaisante, notamment au regard du cathéter décrit dans le document WO 94/04216.

Dans ces conditions, la présente invention a pour but de résoudre le problème technique consistant en la fourniture d'un cathéter de dilatation à échange rapide, d'une nouvelle conception, pouvant être réalisée facilement à l'échelle industrielle, dont la mise en oeuvre est aisée, et permettant de transmettre efficacement jusqu'à la partie distale comportant le ballon, la poussée exercée au niveau de la partie proximale.

La solution conforme à la présente invention, pour résoudre ce problème technique consiste en un cathéter de dilatation à échange rapide comprenant :
- un corps tubulaire flexible comprenant une partie distale, une partie intermédiaire et une partie proximale, et comportant :
- une portion déformable radialement formant ballon, disposée au niveau de sa partie distale ;
- un premier conduit interne débouchant à une extrémité à l'intérieur du ballon, de façon étanche, et reliée à l'autre extrémité à une source d'alimentation en fluide pour permettre le gonflage et le dégonflage du ballon ;
- un second conduit interne, non communiquant avec ledit premier conduit interne, traversant ladite partie distale, et comportant une ouverture au niveau de la partie intermédiaire et au voisinage de la partie proximale, ledit conduit étant défini par une paroi sensiblement tubulaire et adapté pour permettre le passage d'un fil-guide sortant dudit second conduit interne par ladite ouverture, ledit corps comportant en outre une âme présentant un module élastique d'au moins 10000 Mpa reliée fixement à une extrémité à la partie proximale et noyée à son autre extrémité dans la paroi précitée définissant ledit second conduit interne, caractérisé en ce que ladite âme s'étend au-delà de ladite ouverture en direction de l'extrémité distale dudit cathéter.

Ainsi, l'originalité de la présente invention réside dans l'utilisation d'un élément destiné à rigidifier le corps de cathéter, et en particulier au moins une partie de la paroi délimitant le conduit de passage du fil-guide, et d'assurer une transmission sûre et efficace de la poussée exercée au niveau de la partie proximale du cathéter au delà de l'ouverture par laquelle le fil-guide sort dudit conduit de passage et de préférence jusqu'au niveau de sa partie distale.

L'âme étant noyée dans la paroi du second conduit interne précité, il n'y a pas de diminution notable de la section de passage du fluide servant au gonflage du ballon. Cette conformation ne réduit donc d'aucune façon le temps d'inflation et de déflation du ballon.

Selon une caractéristique particulière d'un mode de réalisation, l'âme précitée présente une section transversale qui décroit de son extrémité proximale vers son extrémité distale.

La diminution progressive de la section transversale de l'âme confère donc à l'ensemble du cathéter une progressivité de souplesse en flexion et évite tout risque de cassure ou de plicature de celui-ci.

Le cathéter ainsi obtenu présente une rigidité relativement importante en partie proximale, et une certaine souplesse en partie distale, le passage de la partie rigide à la partie plus souple se faisant sans cassure.

En d'autres termes, le cathéter conforme à la présente invention comporte un corps suffisamment rigide pour permettre une bonne transmission de la poussée exercée sur la partie proximale vers la partie distale tout en assurant une flexibilité suffisante au niveau de la partie distale permettant une maniabilité aisée du cathéter notamment au niveau des courbures du canal corporel.

L'expression "à haut module élastique" utilisée dans le cadre de la présente description et des revendications, vise à couvrir tout matériau présentant un module élastique d'au moins 10000 MPa.

Avantageusement, l'âme précitée sera réalisée en un matériau métallique, de préférence en acier.

Selon une autre caractéristique privilegiée de l'invention, l'âme précitée s'étend dans la partie intermédiaire de préférence sensiblement parallèlement au fil-guide en position dans le second conduit interne, jusqu'à un point situé en amont et au voisinage immédiat de la portion formant ballon.

Le terme amont utilisé ici désigne la partie extérieure au ballon du côté proximal.

Cette conformation particulière permet d'éviter tout risque de perçage du ballon par l'âme précitée tout en assurant la transmission de la poussée jusqu'à la sténose.

Selon un mode de réalisation particulier, le corps tubulaire flexible d'un cathéter conforme à la présente invention comprend :
- une partie proximale, constituée par un tube creux (18) dont l'extrémité distale est légèrement écrasée ;
- une partie distale comprenant un conduit interne sensiblement axial formant la partie distale du second conduit interne précité et un tube externe entourant de façon coaxiale ledit tube interne et comportant une portion déformable radialement formant ballon;
- une partie intermédiaire réalisant une jonction étanche entre les parties proximale et distale comprenant un tube externe prolongeant le tube externe précité de la partie distale et relié à son extrémité proximale à un tube double voies dont la partie distale a été étirée pour former un conduit interne prolongeant de façon étanche le conduit interne de la partie distale précitée.

L'invention sera mieux comprise, et d'autres buts, caractéristiques et avantages de celle-ci apparaîtront plus clairement à la lecture de la description explicative qui va suivre, faite en référence aux dessins schématiques annexés donnés uniquement à titre d'exemples non limitatifs illustrant un mode de réalisation actuellement préféré de l'invention, et dans lesquels :
- la figure 1 est une vue en coupe longitudinale d'un cathéter de dilatation à échange rapide conforme à la présente invention ;
- la figure 2 est une vue en coupe transversale selon la ligne II-II de la figure 1 ;
- la figure 3 est une vue en coupe transversale selon la ligne III-III de la figure 1 ;
- la figure 4 est une vue en coupe transversale selon la ligne IV-IV de la figure 1 ;
- la figure 5 est une vue en coupe transversale selon la ligne V-V de la figure 1 ;
- la figure 6 est une vue de détail en coupe longitudinale montrant la portion sensiblement centrale de la partie intermédiaire d'un cathéter de dilatation conforme à la présente invention ; et
- la figure 7 est une vue de détail en coupe longitudinale montrant la jonction entre les parties intermédiaire et proximale d'un cathéter de dilation conforme à la présente invention.

Dans la description qui va suivre, le canal corporel choisi à titre d'exemple est un vaisseau sanguin non représenté, comme en particulier une artère coronaire.

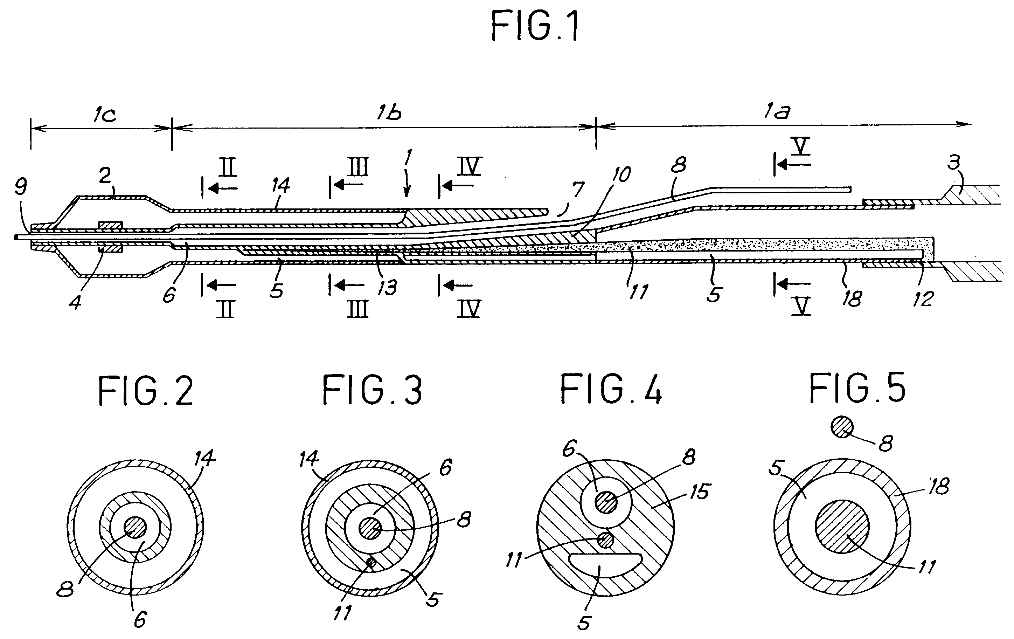

On a donc représenté schématiquement à la figure 1 un cathéter de dilation à échange rapide conforme à la présente invention.

Ce cathéter comprend un corps tubulaire flexible 1 comprenant une partie proximale 1a, une partie intermédiaire 1b et une partie distale 1c.

A titre d'exemple, pour un cathéter ayant une longueur totale d'environ 135 cm, la partie proximale 1a peut présenter une longueur d'environ 111 cm, la partie intermédiaire 1b peut présenter une longueur d'environ 21 cm et la partie distale 1c peut présenter une longueur d'environ 3 cm.

De préférence, le corps tubulaire flexible 1 présentera une section transversale sensiblement circulaire et constante sur la majeure partie de sa longueur.

Le corps 1 présente au niveau de sa partie distale 1c une portion déformable radialement formant ballon 2 et au niveau de sa partie proximale 1a un élément connecteur 3.

Cet élément connecteur 3 est un élément classique à une voie permettant le raccordement à une source d'alimentation en fluide pour permettre le gonflage et le dégonflage du ballon 2.

Le corps flexible 1 peut être réalisé par exemple en une ou plusieurs matières thermoplastiques semi-rigides choisies parmi les polyéthylènes, les polyamides ou bien encore des copolymères de type PEBAX® ou HYTREL®.

La portion déformable radialement formant ballon 2 peut être intégrée au corps flexible 1, comme dans l'exemple représenté, ou rapporté sur celui-ci en y étant fixée de façon étanche par des moyens connus comme par exemple par thermosoudure ou à l'aide d'un adhésif. Cette portion pourra être réalisée également en une matière thermoplastique comme notamment un polyamide, un polyéthylène ou bien encore un polyester.

Dans les dessins, on a représenté la partie formant ballon à l'état gonflé.

Pour faciliter le positionnement du ballon 2 au niveau de la sténose (avant gonflage), le corps 1 pourra être équipé d'un moyen de repérage comme par exemple une bague métallique radio-opaque 4 ; un métal tel que l'or, le platine, le tungstène ou leurs alliages pouvant être utilisés pour la réalisation de cette bague radio-opaque.

D'une façon générale, le corps flexible 1 comprend un premier conduit interne 5 s'étendant sensiblement longitudinalement, ce conduit 5 débouchant à son extrêmité distale à l'intérieur du ballon 2, de façon étanche, et étant relié à son extrémité proximale par l'intermédiaire de l'élément connecteur 3 à une source d'alimentation en fluide non représentée pour permettre le gonflage et le dégonflage du ballon 2.

Le corps flexible 1 comprend également un second conduit interne 6, non communiquant avec le premier conduit interne 5, traversant la partie distale 1c, et se prolongeant au niveau de la partie intermédiaire 1b pour déboucher directement à l'extérieur dudit corps par une ouverture 7 située en aval et au voisinnage de la partie proximale 1a.

Le second conduit interne 6 est défini par une paroi sensiblement tubulaire qui sera décrite plus en détail ci-après, et est adapté pour permettre le passage d'un fil-guide 8 débouchant à l'extrémité distale du cathéter par une ouverture 9 prévue à cet effet et sortant dudit second conduit interne par l'ouverture 7. Le fil-guide 8 habituellement métallique peut être introduit dans le cathéter par saisie de son extrêmité proximale et enfilement dans le conduit interne 6 à partir de l'ouverture distale 9 et par avancement jusqu'à l'ouverture proximale 7 de laquelle il ressort à l'extérieur dudit conduit intene 6 et du cathéter.

Avantageusement, la paroi formant le second conduit interne 6 présentera au niveau de l'ouverture proximale 7 la forme d'une rampe destinée à guider le fil 8 vers l'extérieur du cathéter.

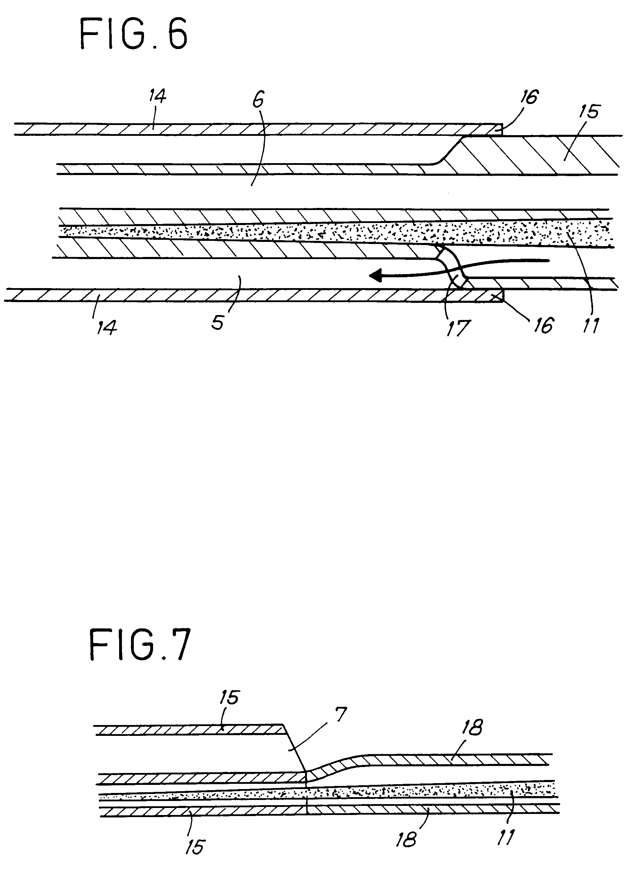

Dans le mode de réalisation actuellement préféré et représenté à la figure 1, les conduits internes 5 et 6 s'étendent sensiblement longitudinalement à l'intérieur du corps 1 et sont coaxiaux dans les parties intermédiaire 1b et distale 1c. Les parties proximale 1a et 1c sont reliées entre elles de façon étanche par la partie intermédiaire 1b, comme il sera expliqué plus en détail en référence aux figures 6 et 7.

Le corps 1 comporte en outre une âme 11 à haut module élastique qui est reliée fixement à une extrémité 12 à la partie proximale 1a et noyé à son autre extrémité 13 dans la paroi définissant le second conduit interne 6 au niveau de la partie intermédiaire 1b, au delà de l'ouverture 7 en direction de l'extrémité distale dudit cathéter.

Ainsi, l'âme 11 peut s'étendre sur une majeure portion de la partie intermédiaire jusqu'à un point situé au voisinage immédiat de la portion formant ballon, ce qui garantit une transmission efficace de la poussée exercée en partie proximale jusqu'à la partie distale.

Avantageusement, la liaison de l'âme 11 à l'extrémité proximale du cathéter se fait au niveau de l'élément connecteur 3, par exemple par collage ou soudure sur ce dernier.

De préférence, l'extrémité 12 de l'âme 11 sera pliée en U afin de lier l'âme 11 à l'élément connecteur 3.

L'âme 11 présente une section transversale par exemple circulaire, qui décroît de son extrémité proximale 12 vers son extrémité distale 13.

L'extrémité distale 13 se trouve de préférence au voisinnage immédiat et en amont de la portion formant ballon 2 précitée.

En référence aux figures 6 et 7 on décrira plus en détail la partie intermédiaire d'un cathéter de dilatation conforme à la présente invention.

Comme on peut le voir sur la figure 6, la paroi déformable radialement formant ballon 2 de la partie distale 1c est prolongée par un tube externe 14 qui s'étend sur une portion de la partie intermédiaire 1b.

Le second conduit interne 6 qui est coaxial relativement au tube externe 14 est obtenu en étirant du côté distal un tube multivoies 15, prolongeant ledit tube externe 14 du côté proximal.

Dans l'exemple représenté, le tube 15 est un tube double voies.

Comme le montre la figure 4, ce tube 15 comporte dans sa partie supérieure une voie à section transversale sensiblement circulaire qui, une fois étirée constitue le second conduit interne 6 de passage du fil guide 8, et dans sa partie inférieure une seconde voie présentant une section transversale sensiblement en forme de "croissant de lune" ou de haricot qui disparaît progressivement par étirage (voir figure 3).

L'âme métallique 11 est disposée dans la seconde voie, et comme on le comprend, reste noyée au niveau de son extrémité distale dans la paroi du tube constituant le second conduit interne 6 après étirage, de préférence sur une majeure portion de la partie intermédiaire.

Le tube externe 14 est fixé en son extrémité proximale 16 au tube double voies 15, de préférence par thermosoudure.

Une ouverture 17 est pratiquée au niveau du rétrécissement dans la partie inférieure du tube double voies 15 afin de constituer ainsi une voie de passage pour le fluide destiné au gonflage et au dégonflage du ballon.

En référence à la figure 7, on décrira maintenant plus en détail la jonction entre les parties intermédiaire 1b et proximale 1a d'un cathéter de dilatation conforme à la présente invention.

La partie proximale 1a est constituée par un tube creux 18 à l'intérieur duquel est disposée l'âme 11.

Pour permettre la jonction avec le tube double voies 15 précité, l'extrémité distale du tube 18 est légèrement écrasée pour constituer une voie dont la section transversale est sensiblement identique à celle de la voie inférieure précitée du tube 15.

La jonction se fait par une mise en contact et soudure des tubes 15 et 18 préalablement disposés de façon à mettre en coincidence la voie inférieure du tube double voies 15 et la voie formée à l'extrémité distale préalablement écrasée du tube 18. La jonction ainsi obtenue est étanche.

L'ouverture 7 précitée est réalisée par découpe de l'extrêmité proximale du tube double voies 15, la partie supérieure écrasée du tube 18 constituant la rampe précitée de guidage du fil 8 vers l'extérieur du cathéter.

Le fonctionnement et l'utilisation du cathéter de dilatation à échange rapide qui vient d'être décrit est conforme à ceux décrits dans l'état de la technique auquel l'homme de métier pourra se reporter.

D'une façon générale, un cathéter de guidage sera tout d'abord introduit dans le vaisseau du patient.

Ensuite, un cathéter de dilation conforme à la présente invention, de dimension convenable prélablement sélectionnée et un fil de guidage 8 seront introduits dans ce cathéter de guidage par avance initiale du fil de guidage jusqu'à la sténose, puis par avance du cathéter de dilatation jusqu'à ce que le ballon 2 se trouve en vis-à-vis de la sténose.

Si l'on souhaite remplacer le cathéter de dilation par un cathéter différent, il suffit de maintenir le fil de guidage 8 en position dans la sténose et de retirer le cathéter par glissement le long du fil 8.

Lorsque le cathéter est séparé du fil de guidage 8, un autre cathéter peut être enfilé sur le fil 8 et avancé jusqu'à la sténose.

## Revendications

1. Cathéter de dilatation à échange rapide, destiné à être introduit dans un canal corporel tel que notamment un vaisseau sanguin, comprenant :
- un corps tubulaire flexible (1) comprenant une partie distale (1c), une partie intermédiaire (1b) et une partie proximale (1a), et comportant :
- une portion déformable radialement formant ballon (2), disposée au niveau de sa partie distale ;
- un premier conduit interne (5) débouchant à une extrémité à l'intérieur du ballon, de façon étanche, et reliée à l'autre extrémité à une source d'alimentation en fluide pour permettre le gonflage et le dégonflage du ballon ;
- un second conduit interne (6), non communiquant avec ledit premier conduit interne (5), traversant ladite partie distale, et comportant une ouverture (7) au niveau de la partie intermédiaire et au voisinage de la partie proximale, ledit conduit étant défini par une paroi sensiblement tubulaire et adapté pour permettre le passage d'un fil-guide (8) sortant dudit second conduit interne (6) par ladite ouverture (7), ledit corps comportant en outre une âme (11) présentant un module élastique d'au moins 10000 Mpa reliée fixement à une extrémité (12) à la partie proximale et noyée à son autre extrémité (13) dans la paroi précitée définissant ledit second conduit interne, caractérisé en ce que ladite âme s'étend au-delà de ladite ouverture (7) en direction de l'extrémité distale dudit cathéter.

2. Cathéter de dilatation à échange rapide selon la revendication 1, caractérisé en ce que l'âme (11) précitée présente une section transversale qui décroit de son extrémité proximale vers son extrémité distale.

3. Cathéter de dilatation à échange rapide, selon la revendication 1 ou 2, caractérisé en ce que l'âme (11) précitée est métallique, notamment en acier.

4. Cathéter de dilatation à échange rapide selon l'une des revendications 1 à 3, caractérisé en ce que l'âme (11) précitée s'étend dans la partie intermédiaire jusqu'à un point situé au voisinage immédiat de la portion formant ballon.

5. Cathéter de dilatation à échange rapide selon l'une des revendications 1 à 4, caractérisé en ce que le second conduit interne (6) précité présente, au niveau de son extrémité débouchant à l'intérieur du cathéter, la forme d'une rampe destinée à guider le fil (8) vers l'extérieur du cathéter.

6. Cathéter de dilatation selon l'une des revendications 1 à 5, caractérisé en ce que le corps tubulaire flexible (1) précité comprend :
- une partie proximale (1a), constituée par un tube creux (18) dont l'extrémité distale est légèrement écrasée ;
- une partie distale (1c) comprenant un conduit interne sensiblement axial formant la partie distale du second conduit interne (6) précité et un tube externe entourant de façon coaxiale ledit tube interne et comportant une portion déformable radialement formant ballon (2) ;
- une partie intermédiaire (1b) réalisant une jonction étanche entre les parties proximale (1a) et distale (1c) comprenant un tube externe (14) prolongeant le tube externe précité de la partie distale et relié à son extrémité proximale à un tube double voies (15) dont la partie distale a été étirée pour former un conduit interne prolongeant de façon étanche le conduit interne de la partie distale précitée.

7. Cathéter de dilatation à échange rapide selon l'une des revendications précédentes, caractérisé en ce que l'âme (11) précitée comporte une extrémité proximale pliée en U.

## Claims

1. A rapid-exchange dilatation catheter, for introduction into a body canal notably such as a blood vessel, comprising:
- a flexible tubular body (1) comprising a distal part (1c), an intermediate part (1b) and a proximal part (1a), and having:
- a radially deformable portion forming balloon (2) disposed at the level of its distal part;
- a first inner duct (5) issuing at one end inside the balloon, in a liquid-tight manner, and connected at the other end to a fluid supply source in order to enable inflating and deflating of the balloon;
- a second inner duct (6), which is not communicating with said first inner duct (5), traversing said distal part and comprising an opening (7) at the level of the intermediate part and close to the proximal part, said duct being defined by a substantially tubular wall and adapted to allow the passage of a guide-wire (8) coming out from said second inner duct (6) through said opening (7), said body further comprising a core (11) having a high modulus of elasticity of at least 10000 Mpa and being permanently joined at one end (12) to the proximal part and embedded at its other end (13) in the above-mentioned wall defining said second inner duct, characterised in that said core extends beyond said opening (7) in the direction of the distal end of said catheter.

2. The rapid-exchange dilatation catheter according to claim 1, characterised in that the above-mentioned core (11) has a cross-section which is decreasing from its proximal end towards its distal end.

3. The rapid-exchange dilatation catheter according to claim 1 or 2, characterised in that the above-mentioned core (11) is in metal, preferably steel.

4. The rapid-exchange dilatation catheter according to one of claims 1 to 3, characterised in that the above-mentioned core (11) extends in the intermediate part up to a point situated in the immediate vicinity of the balloon-forming portion.

5. The rapid-exchange dilatation catheter according to one of claims 1 to 4, characterised in that the above-mentioned second inner duct (6) has, at the level of its end issuing into the catheter, the shape of a ramp intended for guiding the wire (8) out of the catheter.

6. The dilatation catheter according to one of claims 1 to 5, characterised in that the above-mentioned flexible tubular body (1) comprises:
- a proximal part (1a) constituted by a hollow tube (18) of which the distal end is slightly flattened;
- a distal part (1c) comprising a substantially axial inner duct forming the distal part of the above-mentioned second inner duct (6) and an outer tube surrounding coaxially said inner tube and comprising a radially deformable portion forming balloon (2);
- an intermediate part (1b) forming a liquid-tight connection between the proximal part (1a) and the distal part (1c), comprising an outer tube (14) extending the above-mentioned outer tube of the distal part and being joined at its proximal end to a two-channel tube (15) whose distal part has been stretched in order to form an inner duct extending in a liquid-tight manner the inner duct of the above-mentioned distal part.

7. The rapid-exchange dilatation catheter according to one of the preceding claims, characterised in that the above-mentioned core (11) has a proximal end bent in the form of a U.

## Patentansprüche

1. Dilatationskatheter zum raschen Wechseln, der dazu bestimmt ist, in einen Körperkanal, wie insbesondere ein Blutgefäß, eingeführt zu werden, mit:
- einem flexiblen rohrförmigen Körper (1), der einen distalen Teil (1c), einen Zwischenteil (1b) und einen proximalen Teil (1a) enthalt und:
- einen radial verformbaren Teil, der einen Ballon (2) bildet, der auf der Höhe seines distalen Teils angeordnet ist;
- eine erste innere Leitung (5), die an einem Ende abgedichtet in das Innere des Ballons mündet, und am anderen Ende mit einer Fluid-Zufuhrquelle verbunden ist, um das Aufblasen und Ablassen des Ballons zu ermöglichen;
- eine zweite innere Leitung (6) aufweist, die mit der ersten inneren Leitung (5) nicht in Verbindung steht, den distalen Teil durchquert und eine Öffnung (7) auf der Höhe des Zwischenteils und in der Nähe des proximalen Teils hat, welche Leitung durch eine im wesentlichen rohrförmige Wand definiert und eingerichtet ist, den Durchgang eines Führungsdrahts (8) zu ermöglichen, der aus der zweiten inneren Leitung (6) durch die Öffnung (7) austritt, welcher Körper außerdem einen Kern (11) aufweist, der einen Elastizitsmodul von zumindest 10 000 MPa hat, fest an einem Ende (12) mit dem proximalen Teil verbunden ist, und an seinem anderen Ende (13) in der die zweite innere Leitung definierenden Wand aufgenommen ist, dadurch gekennzeichnet, daß sich der Kern über die Öffnung (7) hinaus in der Richtung des distalen Endes des Katheters erstreckt.

2. Dilatationskatheter zum raschen Wechseln nach Anspruch 1, dadurch gekennzeichnet, daß der Kern (11) einen Querschnitt aufweist, der von seinem proximalen Ende zu seinem distalen Ende abnimmt.

3. Dilatationskatheter zum raschen Wechseln nach Anspruch 1 oder 2, dadurch gekennzeichnet, daß der Kern (11) aus einem Metall, insbesondere aus Stahl, besteht.

4. Dilatationskatheter zum raschen Wechseln nach einem der Ansprüche 1 bis 3, dadurch gekennzeichnet, daß sich der Kern (11) im Zwischenteil bis zu einem Punkt in unmittelbarer Nähe des den Ballon bildenden Teils erstreckt.

5. Dilatationskatheter zum raschen Wechseln nach einem der Ansprüche 1 bis 4, dadurch gekennzeichnet, daß die zweite innere Leitung (6) auf der Höhe ihres in das Innere des Katheters mündenden Endes die Form einer Rampe aufweist, die dazu bestimmt ist, den Draht (8) zur Außenseite des Katheters zu führen.

6. Dilatationskatheter zum raschen Wechseln nach einem der Ansprüche 1 bis 5, dadurch gekennzeichnet, daß der flexible rohrförmige Körper (1):
- einen proximalen Teil (1a), der aus einem hohlen Rohr (18) besteht, dessen distales Ende leicht zusammengedrückt ist;
- einen distalen Teil (1c), der eine im wesentlichen axiale innere Leitung hat, die den distalen Teil der zweiten inneren Leitung (6) bildet, und ein Außenrohr, das koaxial das Innenrohr umgibt und einen radial verformbaren Teil hat, der einen Ballon (2) bildet;
- einen Zwischenteil (1b) aufweist, der eine dichte Verbindung zwischen dem proximalen (1a) und distalen (1c) Teil bewirkt, mit einem Außenrohr (14), das das Außenrohr des distalen Teils verlängert und an seinem proximalen Ende mit einem Zweiwegrohr (15) verbunden ist, dessen distaler Teil gestreckt ist, um eine innere Leitung zu bilden, die abdichtend die innere Leitung des distalen Teils verlängert.

7. Dilatationskathether zum raschen Wechseln nach einem der vorhergehenden Ansprüche, dadurch gekennzeichnet, daß der Kern (11) ein in Form eines U gefaltetes proximales Ende aufweist.
